# EUROPEAN PATENT APPLICATION

(11) **EP 1 878 798 A1**
(43) Date of publication of application: **16.01.2008**
(21) Application number: 06291150.8
(22) Date of filing: 13.07.2006
(51) Int. Cl.: C12N 15/85, A01K 67/027, G01N 33/50

(54) **Method of producing a multichimeric mouse and applications to study the immunopathogenesis of human tissue-specific pathologies**

(71) Applicant: INSTITUT PASTEUR, 75015 Paris (FR)
(72) Inventor: Garcia, Sylvie, 94230 Cachan (FR); Tartour, Eric, 75003 Paris (FR)
(74) Representative: Goulard, Sophie

(57) **Abstract**

The invention relates to a method of producing a multichimeric mouse comprising a functional xenogenic (human) immune system restricted to the MHC class I and/or class II molecules (HLA molecules) of the xenogenic species solely, and a functional syngenic tissue. The invention relates also to the use of the multichimeric mouse obtainable by said method, to study the immunopathogenesis of tissue-specific diseases (infectious, tumoral or auto-immune pathologies) and to their applications to design and test vaccines or immunotherapeutic agents against these pathologies.

## Description

The invention relates to a method of producing a multichimeric mouse comprising a functional xenogenic (human) immune system restricted to the MHC class I and/or class II molecules (HLA molecules) of the xenogenic species solely, and a functional syngenic tissue. The invention relates also to the use of the multichimeric mouse obtainable by said method, to study the immunopathogenesis of tissue-specific diseases (infectious, tumoral or auto-immune pathologies) and to their applications to design and test vaccines or immunotherapeutic agents against these pathologies.

Animal models having the components and complexity of a living organism which are missing in human cells *in vitro* assays, are essential to design appropriate preventive and curative therapies (vaccination, immunotherapy) against human diseases. However, few animal models are available for the pathologies which are strictly restricted to humans. In addition, the existing models are expensive, difficult to set up (simian models of HIV infection) or distantly related to the human pathology (mice model of malaria or shigellosis).

To overcome these limitations transgenic mice expressing human molecules (CD4, chimiokine co-receptor, HLA) have been constructed. However, these transgenic models allow the study of a specific step only of the disease, which do not reflect the complexity of the human organism response to the disease.

Small animal xenotransplantation models trying to reproduce human hematopoiesis, have been employed in order to analyze human immune system development and function *in vivo.* In these models, human hematopoietic cells and tissues are transplanted into mice that are compromised in their capacity to reject xenografts, so as to generate human/mouse chimera or humanized mice.

Engraftment was first reported after transfer of mature human peripheral blood leukocytes in severe combined immunodeficient mice (huPBL-SCID mice; Mosier et al., Nature, 1998, 335, 256-259) and transplantation of blood-forming fetal liver cells, fetal bone, fetal thymus and fetal lymph nodes in SCID mice (SCID-hu mice; McCune et al., Science, 1988, 241, 1632-1639; McCune et al., Semin. Immunol., 1996, 8, 187-). After these initial reports in T and B lymphocyte-deficient SCID mice (Prkdc^{scid} mutant mice), some level of engraftment was also achieved by transplantation of blood-forming cells in recombination activating gene (RAG)-deficient mice (Rag1^{-/-}, Rag2^{-/-} mutant mice; Schultz et al., J. Immunol., 2000, 164, 2496-2507; Goldman et al., Br. J. Haematol., 1998, 103, 335-342). The engraftment levels in these models, however, were still low presumably due to the remaining innate immunity of host animals. Nonobese diabetic/severe combined immunodeficient (NOD/SCID) mice have been shown to support higher levels of human progenitor cells engraftment than BALB/c/SCID or C.B.17/SCID mice (Greiner et al., Stem Cells, 1998, 16, 166-). NOD/SCID mice harboring either a null allele at the beta-2 microglobulin gene (NOD/SCID/β2m^{-/-}; O. Kollet et al., Blood, 2000, 95, 3102-) or a truncated common cytokine receptor γ chain (γc) mutant lacking its cytoplasmic region (NOD/SCID/γ_{c}^{-/-}; Ito et al., Blood, 2002, 100, 3175-) were developed. In these mice, NK-as well as T- and B-cell development and functions are disrupted, because β2m is necessary for major histocompatibility complex (MHC) class 1-mediated innate immunity, and because γc (originally called IL-2Rγ chain) is an indispensable component of receptor heterodimers for many lymphoid-related cytokines (IL-2, IL-7, IL-9, IL-12, IL-15 and IL-21), whose some are required for generation/maintenance of lymphoid lineages.. However, these models sustain only limited development and maintenance of human lymphoid cells and rarely produce immune responses.

More recently, intrahepatic injection of CD34⁺ human cord blood cells into irradiated newborn Rag2^{-/-}/γ_{c}^{-/-} mice led to the formation of a quantitatively and functionally complete immune system, as demonstrated by *de novo* development of B, T, and dendritic cells; formation of structured primary and secondary lymphoid organs; and production of functional immune response (Traggai et al., Science, 2004, 304, 104-107). The efficiency of this xenogenic transplantation system was further confirmed in the same mouse strain (Gimeno et al., Blood, 2004, 104, 3886-3883) as well as in NOD/SCID mice harboring a complete null mutation of the common cytokine receptor γ chain (NOD/SCID/γ_{c}^{null}; Ishikawa et al., Blood, 2005, 106, 1565-1573).

Nevertheless, this model presents several disadvantages: it is restricted to the hematopoietic tissue solely; the quantitative T cell reconstitution is very poor, with a very limited number of T cells in the chimera lymphoid organs. Furthermore, functional studies are difficult in this system since the nature of the antigen presenting cells (murine *versus* human) remains unclear and do not allow to exclude the possible restriction of human T cell responses by murine MHC.

To overcome these limitations, the present invention provides a method of producing a multichimeric mouse, characterized in that it comprises the step of: transplanting syngenic precursor cells of a xenogenic species into a transgenic mouse, by any appropriate means, wherein:
a) the syngenic precursor cells comprise hematopoietic and non-hematopoietic precursors, and
b) the transgenic mouse has a phenotype comprising :
   b₁) a deficiency for murine T lymphocytes, B lymphocytes and NK cells,
   b₂) a deficiency for murine MHC class I and MHC class II molecules, and
   b₃) a functional MHC class I transgene and/or a functional MHC class II transgene from the same species as the precursor cells.

The substitution of the host murine MHC molecules by HLA molecules, in the transgenic mice according to the present invention, improves considerably the initial models. In a HLA context, the human CD4/MHC class II and CD8/MHC class I interactions which are stronger than the xenogenic interactions mainly encountered in the previous models increase drastically the T cell number both by facilitating positive selection of human thymocytes and peripheral survival of generated human T cells. In addition, the substitution of the host murine MHC molecules by HLA molecules ensures the restriction of human T cell response in a human MHC context. The haplotype of the murine host should not be obligatory the one of the donor cells to ensure the previous functions. If the HLA haplotype is different between the murine hosts and the donor cells, the donor T cells will be educated in both host and its HLA context. Mice of the invention are used as recipient hosts for human hematopoietic and non-hematopoietic precursors transplantation, to generate new human/mouse multichimera. The multichimeric mouse comprises a functional xenogenic (human) immune system restricted to the MHC class I and/or class II molecules (HLA molecules) of the xenogenic species solely, and at least one functional syngenic tissue.

These mice represent a completely humanized model, cheap, easy to set up, reproducible, flexible (HLA variability/tissue variety), that can be used to study the immunopathogenesis of wide range of tissue-specific diseases (infectious, tumoral and auto-immune pathologies), as well as the role of the immune system in tissue differenciation *in vivo.* These mice provide a model useful in the development and optimization of vaccines or immunotherapies with maximum efficacy *in vivo* for human use. Specifically, such mice enable a complete analysis, in a single animal, of the components of the immune adaptative response (antibody, helper and cytolytic) which are elicited against the antigens which are expressed in a human tissue which is affected by a wide range of pathologies (infectious disease, cancer, auto-immune disease). In this model it is possible to identify the effectors of the immune response and the antigens they are directed to (antigen-specific antibodies, antigen specific HLA class II restricted CD4⁺ T cells, antigen specific HLA class I restricted CD8⁺ T cells). It is also possible to study how these responses cooperate. For example, a defined sub-population is depleted by anti-CD antibodies treatment (anti-CD4, CD8, NK, CD19, CD25...), in order to evaluate its role. It is easy to manipulate the genetic content of the precursor cells to study the involvement of a defined gene. The possibility to have access to a large panel of different HLA allotypes reflective of the genetic variability of the human population either for the murine hosts and/or for the donor precursor cells (estimated in Taylor et al, The Lancet, 2005, 366:2019-2025) allows the study to cover the overall human population. Once, the effectors which are elicited and the antigens they recognize have been identified, it is then possible to design appropriate immunotherapeutic agents to control the immune response and appropriate vaccines to induce a protective immune response, and thus to prevent or treat the tissue-specific disease. Therefore, this model is useful to set up more efficient prophylactic or curative immunotherapies and vaccines against human pathogens, cancer and auto-immune diseases. These mice represent an optimized tool for basic and applied immunology studies.

### Definitions

- "xenogenic", "xenogenic species" refers to a non-mouse vertebrate.
- "syngenic cells" refers to cells from individuals with the same genotype (cells from a unique individual or from identical twins).
- "stem cell" refers to pluripotent or multipotent cell having clonogenic and self-renewing capabilities and the potential to differentiate into multiple cell lineages. These cells allow the reconstitution of multiple somatic tissue types.
- "precursor", "precursor cell" refers to committed cell having the potential to differentiate into a particular cell lineage. These cells allow the reconstitution of a specific somatic tissue type.
- "chimeric" or "chimera" refers to xenograft of cells transplanted from one species into a host of another species.
- "multichimeric" or "multichimera" refers to xenograft of at least two different cell types, transplanted from one species into a host of another species.
- "deficiency for" refers to the lack of a molecular or cellular function.
- "mutation"refers to the substitution, insertion, deletion of one or more nucleotides in a polynucleotide sequence.
- "deficient gene", "inactivated gene", "null allele" refers to a gene comprising a spontaneous or targeted mutation that results in an altered gene product lacking the molecular function of the wild-type gene.
- "disrupted gene" refers to a gene that has been inactivated using homologous recombination or other approaches known in the art.
- "transgene" refers to a nucleic acid sequence, which is partly or entirely heterologous, i.e., foreign, to the transgenic animal or cell into which it is introduced, or, is homologous to an endogenous gene of the transgenic animal or cell into which it is introduced, but which is designed to be inserted, or is inserted, into the animal's genome in such a way as to alter the genome of the cell into which it is inserted (e.g., it is inserted at a location which differs from that of the natural gene or its insertion results in a knockout). A transgene can be operably linked to one or more transcriptional regulatory sequences and any other nucleic acid, such as introns, that may be necessary for optimal expression of a selected nucleic acid.
- "functional transgene" refers to a transgene that produces an mRNA transcript, which in turn produces a properly processed protein in at least one cell of the mouse comprising the transgene. One of skill will realize that the diverse set of known transcriptional regulatory elements and sequences directing post-transcriptional processing provide a library of options from which to direct the expression of a transgene is a host mouse. In many embodiments of the invention, expression of an HLA transgene under the control of an H-2 gene regulatory element may be preferred.
- "HLA" refers to the human MHC complex and "H-2" to the mouse MHC complex.

According to the method of the present invention, the hematopoietic precursors and non-hematopoietic precursors may be isolated from appropriate tissues (fetal tissue, cord-blood, adult bone-marrow) or they may be derived *in vitro,* from adult or embryonic stem cells, by methods which are well-known to those of ordinary skill in the art. For example, methods for differentiating human embryonic stem cells in multiple different lineages, *in vitro,* are described in Hyslop et al., Expert. Rev. mol. Med., 2005, 7, 1-21; Odorico et al., Stem cells, 2001, 19, 193-204. Methods for differentiating human embryonic stem cells, specifically in CD34+ cells, hepatic cells, pancreatic cells or neurones are described, respectively, in: Vodyanik et al., Blood, 2005, 105, 617-626; Lavon et al., Differentiation, 2004, 72, 230-238 and Levon and Benvenisty, J. Cell. Bioch., 2005, 96:1193-1202 ; Assady et al., Diabetes, 2001, 50, 1961-1967; Zhang et al., Nat. Biotechnol., 2001, 19, 1129-1133. For example, methods for differentiating human adult stem cells in multiple different or single lineages, *in vitro,* are reviewed in K6rbling et al., N Engl J Med, 2003, 349:570-582.

In a first embodiment, the invention provides a method wherein the precursor cells are derived from stem cells.

The use of stem cells allows to derive more progenitor cells since the source of biological material is available in higher quantity and contains more precursor cells, in particular for the CD34+ hematopoietic precursors. In addition, the embryonic stem cells are less immunogenic. These advantages increase the reproducibility between different mouse chimera obtained by transplantation of the same progenitor cells preparation to different mice of the same transgenic strain.

In another embodiment, the invention provides a method wherein the precursor cells are human precursor cells.

In another embodiment, the invention provides a method wherein the hematopoietic precursor cells are human CD34⁺ cells.

In another embodiment, the invention provides a method wherein the non-hematopoietic precursor cells are selected from the group consisting of: hepatocyte, neurone, adipocyte, myocyte, chondrocyte, or melanocyte precursors, and endothelial, glial, or pancreatic cells precursors.

In another embodiment, the invention provides a method wherein the the hematopoietic precursors or non-hematopoietic precursors are genetically modified by an oligonucleotide or a polynucleotide of interest, so as to induce the expression of a heterologous gene or inhibit the expression of an endogenous gene. The modification may be stable or transient. For example, the cells may be transgenic cells expressing a gene of interest, such as a cytokine gene or an oncogene. For example the transgenic expression of IL-7 or IL-15 involved in the generation/maintenance of T cell memory may be useful to induce efficient vaccination. Precursor cells may be transgenic for the expression of human *c-Ha-ras* gene with its own promoter which promotes further induction of carcinoma after treatment by genotoxic carcinogens like N-ethyl-N-nitrosourea, 7,12-dimethylbenz(a)anthracene (DMBA) or urethane (M. Okamura et al., Cancer letters, 2006: 1-10). The conditional expression of oncogenes like SV40 early sequence under the control of the regulatory sequences of the human antithrombin III gene that confer hepatic expression, may provide good model for hepatic carcinomas (D-Q Lou et al., Cancer letters 2005, 229:107-114). Alternatively, the cells may be transiently modified by a siRNA targeting a gene of interest for example conditional shutting down of the expression of IL-2 gene involved in regulatory T cells maintenance/function may be advantageous for the development of efficient vaccination. The conditional knocking down of pro-apoptotic genes may also be investigated for the occurrence of tumors.

In another embodiment, the invention provides a method wherein the the hematopoietic precursors and non-hematopoietic precursors are from donors of different MHC haplotypes, more preferably of the haplotypes that are the most frequent in the xenogenic species, to take into account the xenogenic MHC polymorphism. For example, the mice are transgenic for the HLA haplotypes that are the most frequent in the human population. These humanized mice have HLA molecules that are reflective of the genetic variability of the human population. Therefore, their immune system is reflective of the immune system of most individuals of the population.

The haplotype of the precursor cells may also correspond to an haplotype that is involved in disease development or outcome, for example auto-immune diseases (Jones et al., Nature Reviews Immunol., 2006, 6:271-282) or viral infections like HCV (Yee L.J., Genes and Immunity, 2004, 5:237-245) or HIV (Bontrop R.E. and D.I. Watkins, Trends in Immunol., 2005, 26:227-233).

In another embodiment, the invention provides a method wherein the the hematopoietic precursors and non-hematopoietic precursors are transplanted simultaneously.

In another embodiment, the invention provides a method wherein the hematopoietic precursors and non-hematopoietic precursors are transplanted sequentially.

In another embodiment, the invention provides a method wherein the hematopoietic precursors and non-hematopoietic precursors are transplanted in the same site of the mouse.

In another embodiment, the invention provides a method wherein the the hematopoietic precursors and non-hematopoietic precursors are transplanted in a different site of the mouse

Methods of transplanting progenitor cells into mice are well-known in the art. The hematopoietic progenitor cells are preferably transplanted into sublethally irradiated newborn mice For example, from 10⁴ to 10⁶ human CD34+ cells are transplanted intraperitoneally, intra-hepatically, or via a facial vein, into sublethally irradiated newborn transgenic mice, as described in Traggiai et al., Science, 2004, 304, 104-107; Ishikawa et al., Blood, 2005, 106, 1565-1573; Gimeno et al., Blood, 2004, 104, 3886-3893.

The hematopoiteic and non-hematopoietic progenitor cells may be transplanted simultaneously or sequentially. Both strategies may be dictated both by scientific or technical reasons. For example, it may be difficult to inject the same day neuronal and hematopoietic precursors into brain and liver of newborn mice, while hepatic and hematopoietic precursors can be injected simultaneously in the liver. Preferably, the transplantation of the hematopoietic tissue is intrahepatic and the transplantation of the non-hematopoietic tissue is orthotopic or not depending on the organ. For example, the hematopoietic/hepatic reconstitution are achieved by transplantating both precursors intrahepatically. The hematopoietic/neuronal reconstitutions are achieved by transplanting the hematopoietic precursors intrahepatically and the non-hematopoietic precursors at the orthotopic site (brain). The hematopoietic/pancreatic reconstitutions are achieved by transplanting the hematopoietic precursors intrahepatically and the pancreatic precursors under the kidney capsules of the murine hosts.

The transgenic mouse as defined in the present invention which is deficient for murine T and B lymphocytes, and NK cells, comprises two genes essential in T, B and/or NK cells development that are inactivated by a spontaneous mutation or a targeted mutation (deficient genes). These mutations which are well-known to those of ordinary skill in the art include, for example: a first mutation which is the mouse *scid* mutation (Prkdc^{scid}; Bosma et al., Nature 1983, 301, 527-530; Bosma et al., Curr. Top. Microbiol., Immunol., 1988, 137, 197-202) or the disruption of the recombination activating gene (Rag.1^{-/-} or Rag2^{-/-}; Mombaerts et al., Cell, 1992, 68, 869-877; Takeda et al., Immunity, 1996, 5, 217-228), and a second mutation which is the beige mutation (Lyst^{bg}; Mac Dougall et al., Cell. Immunol., 1990, 130, 106-117) or the disruption of the β₂-microglobulin gene (β₂m^{-/-}; Kollet et al., Blood, 2000, 95, 3102-3105), the IL-2 receptor γ chain (or common cytokine receptor γ chain (γ_{c})) gene (IL-2Rγ^{-/-} or γ_{c}^{-/-}; DiSanto et al., P.N.A.S., 1995, 92, 377-381), or the IL-2 receptor β chain (IL-2Rβ) gene (IL-2Rβ^{-/-}; Suzuki et al; J. Exp. Med., 1997, 185, 499-505)

In addition these mutations are in an appropriate genetic background which is well-known to those of ordinary skill in the art. For example, the SCID mutation is preferably in a NOD background (diabetes-susceptible Non-obese Diabetic back-ground, NOD/SCID; Prochazka et al., P.N.A.S., 1992, 89, 3290-3294).

For example, the mouse according to the present invention comprises one of the following genotypes corresponding to a T, B and NK cell deficiency : SCID/Beige (*scid*/*scid, bg*/*bg ;* Mac Dougall et al., Cell. Immunol., 1990, 130, 106-117), NOD/SCID/IL2-Rγ^{null} (Ishikawa et al., Blood, 106, 1565-1573; Schultz et al., J. Immunol., 2005, 174, 6477-6489), NOD/SCID/β₂m^{-/-} (Zijlstar et al., Nature, 1990, 344, 742-746), Rag2^{-/-}/γ_{c}^{-/-} (Goldman et al., Br. J. Haematol., 1998, 103, 335-342).

In another embodiment, the invention provides a method wherein the transgenic mouse deficiency as defined in b₁) is associated with a deficient Rag2 gene and a deficient common receptor γ chain gene. Preferably, both genes are disrupted by homologous recombination (Rag2 and γ_{c} knock-out or Rag2^{-/-}/γ_{c}^{-/-}).

The MHC class I molecule comprises an α-chain (heavy chain) which is non-covalently associated with a β2-microglobulin (β2-m) light chain. The MHC class II molecules are heterodimers comprising an α-chain and a β-chain. The human complex comprises three class I α-chain genes: HLA-A, HLA-B, and HLA-C and three pairs of MHC class II α- and β- chain genes, HLA-DR, -DP, and -DQ. In many haplotypes, the HLA-DR cluster contains an extra β-chain gene whose product can pair with the DRα chain, and so the three sets of genes give rise to four types of MHC class II molecules. In the mouse, the three class I α-chain genes are H-2-L, H-2-D, and H-2K. The mouse MHC class II genes are H-2-A and H-2-E. H-2-Eα is a pseudogene in the H2^{b} haplotype.

In another embodiment, the invention provides a method wherein the transgenic mouse deficiency in murine MHC class I molecules is associated with a deficient β2-microglobulin gene, preferably a disrupted β2-microglobulin gene (β₂m knock-out, β₂m^{-/-}); the absence of β2-microglobulin chain in the mouse leads to lack of murine MHC class I molecules (H-2-L, H-2-D, and H-2K) cell surface expression.

In a preferred embodiment, the β2-microglobulin gene and at least one of the class I α-chain genes, for example the H2-D^{b} and/or H2-K^{b} genes, are disrupted.

β₂m knock-out mice are well-known to those of ordinary skill in the art. For example, β2m^{-/-} mice are described in Zijlstar et al., Nature, 1990, 344, 742-746, and β₂m^{-/-}, H2-D^{b} and/or H2-K^{b} mice can be obtained as described in Pascolo et al., J. Exp. Med., 1997, 185:2043-2051.

In another embodiment, the invention provides a method wherein the transgenic mouse deficiency in murine MHC class II molecules is associated with a deficient H-2^{b}-Aβ gene, and eventually a deficient H-2-Eβ gene.

In a H2^{b} haplotype, the deficiency in murine MHC class II molecules is obtained by disrupting the H-2^{b}-Aβ gene (I-Aβ^{b} knock-out or I-Aβ^{b -/-}); the absence of H-2 I-A β-chain leads to lack of conventional H-2 I-A and I-E class II molecules cell surface expression, since H-2-Eα is a pseudogene in this haplotype. In the other H-2 haplotypes, the deficiency in murine MHC class II molecules is obtained by disrupting both H-2-Aβ and H-2-Eβ genes.

I-Aβ^{b} knock-out mice are well-known to those of ordinary skill in the art. For example, I-Aβ^{b -/-} mice are described in Takeda et al., Immunity, 1996, 5, 217-228. Mice knocked out for both H-2-Aβ and H-2-Eβ genes are described in Madsen et al., Proc. Natl. Acad. Sci. USA, 1999, 96:10338-10343.

One of the difficulties hampering the design of T-epitope-based vaccines targeting T lymphocytes is HLA class I/class II molecule polymorphism. It is known in the art that genetic diversity exists between the HLA genes of different individuals as a result of both polymorphic HLA antigens and distinct HLA alleles Due to the high degree of polymorphism of the HLA molecules, the set of epitopes from an antigen, which are presented by two individuals may be different depending on the HLA molecules or HLA type which characterize said individuals. However, despite of a high number of HLA molecules whose repartition is not homogeneous worldwide, some alleles are predominant in human populations (HLA-DR1, -DR3, - B7, -B8, -A1, -A2). For example, HLA-A2.1 and HLA-DR1 molecules are expressed by 30 to 50 % and 6 to 18 % of individuals, respectively. In addition, there is a redundancy of the presented set of peptides between HLA class I isotypic or allelic variants and the binding of peptides to HLA class II molecules is less restrictive than to class I molecules. Therefore, the peptides which are presented by the HLA-A2.1 and HLA-DR1 molecules should be representative of the epitopes that are presented by most individuals of the population. Nethertheless, it may be desirable to identify the optimal epitopes that are presented by other HLA isotypic or allelic variants, to cover the overall human population. This may be also important in cases where there is a bias in the immune response, so that the antigen is preferably presented by other HLA haplotypes. This may be also relevant in cases where the HLA haplotypes are involved in disease development or outcome, for example auto-immune diseases (Jones et al., Nature Reviews Immunol, 2006, 6:271-282) or viral infections like HCV (Yee L.J., Genes and Immunity, 2004, 5:237-245) or HIV (Bontrop R.E. and D.I. Watkins, Trends in Immunol., 2005, 26:227-233).

The transgenic mouse which is used in the method of the present invention may comprise one or more functional xenogenic MHC class I and/or class II transgene(s). Preferably the allotypes of the MHC class I and/or class II transgenes, are reflective of the genetic variability of the xenogenic population. For example, the allotypes which are the most frequent in the xenogenic population are chosen so as to cover the overall xenogenic population. The xenogenic MHC class I and/or class II transgenes may have the sequence of the alleles that are present in the precursor cells or the sequence of other alleles that are not present in the precursor cells.

In another embodiment, the invention provides a method wherein the transgenic mouse xenogenic MHC class I transgene is a human HLA class I transgene and the transgenic mouse xenogenic MHC class II transgene is a human HLA class II transgene.

The human HLA class I and class II transgenes may have the sequence of the alleles that are present in the precursor cells or the sequence of other alleles that are not present in the precursor cells.

Preferably, the human HLA class I and class II transgenes correspond to allotypes that are the most frequent in the human population.

For example, the human HLA class I transgene is an HLA-A2 transgene and the HLA class II transgene is an HLA-DR1 transgene.

More preferably, the HLA-A2 transgene encodes a HLA-A2.1 monochain in which the human β2m is covalently linked by a peptidic arm to the HLA-A2.1 heavy chain.

The HLA-DR1 α and β chains may be encoded by the HLA-DRA*0101 and the HLA-DRB1*0101 genes, respectively.

HLA class I and HLA class II transgenic mice are well-known to those of ordinary skill in the art. For example, HLA-A2.1 transgenic mice expressing a chimeric monochain (α1-α2 domains of HLA-A2.1 (encoded by HLA-A*0201 gene), α3 to cytoplasmic domains of H-2 D^{b}, linked at its N-terminus to the C terminus of human β2m by a 15 amino-acid peptide linker) are described in Pascolo et al., J. Exp. Med., 1997, 185, 2043-2051.

HLA-DR1 transgenic mice expressing the DR1 molecule encoded by the HLA-DRA*0101 and HLA-DRB1*0101 genes are described in Altmann et al., J. Exp. Med., 1995, 181, 867-875.

Accordingly, embodiments of the invention disclosed herein may substitute one polymorphic HLA antigen for another or one HLA allele for another. Examples of HLA polymorphisms and alleles can be found, for example, at http://www.anthonynolan.org.uk/HIG/data.html and http://www.ebi.ac.uk/imgt/hla, and in Genetic diversity of HLA: Functional and Medical Implication, Dominique Charon (Ed.), EDK Medical and Scientific International Publisher, and The HLA FactsBook, Steven G.E. Marsh, Peter Parham and Linda Barber, AP Academic Press, 2000.

The human HLA class I and class II transgenes may also correspond to allotypes that are involved in disease development or outcome, for example auto-immune diseases (Jones et al., Nature Reviews Immunol., 2006, 6:271-282) or viral infections like HCV (Yee L.J., Genes and Immunity, 2004, 5:237-245) or HIV (Bontrop R.E. and D.I.Watkins, Trends in Immunol., 2005, 26:227-233).

Alternatively, MHC class I and class II molecules from other vertebrates can be expressed in the transgenic mice according to the present invention. MHC gene sequences are accessible in the data bases such as the NCBI database (http://www.ncbi.nlm.nih.gov/).

In another embodiment, the invention provides a method wherein the transgenic mouse has one of the following genotypes:
- Rag2^{-/-}, γ_{c}^{-/-}, β₂m^{-/-}, I-Aβ^{b-/-}, HLA-A2^{+/+}, HLA-DR1^{+/+},
- Rag2^{-/-}, γ_{c}^{-/-}, β₂m^{-/-}, I-Aβb^{-/-}, HLA-A2^{+/+}, and
⁻Rag2^{-/-}, γ_{c}^{-/-}, β₂m^{-/-}, I-Aβ^{b-/-}, HLA-DR1^{+/+}.

In another embodiment, the invention provides a method wherein the transgenic mouse, further comprises a deficiency for the C5 protein of complement (C5^{-/-}). The mouse of the strains 129 or FBV are examples of mice having a deficiency for the C5 protein of complement. These strains can advantageously be used for making the transgenic mouse which is used in the present invention.

The invention relates also to the use of a multichimeric mouse obtainable by the method as defined above, to study the role of the immunopathogenesis of a tissue-specific disease.

The invention relates also to the use of a multichimeric mouse obtainable by the method as defined above, to study tissue differentiation *in vivo:* the multichimeric mouse provides a model to study the development of the adaptative immune system and another syngenic tissue of a xenogenic (for example human) species, *in vivo.*

In a preferred embodiment of the preceding uses, the multichimeric mouse comprises:
- functional transgenic-MHC class I and/or MHC class II molecules of the xenogenic species; the MHC class I and class II molecules may correspond to an haplotype which is identical or different to that of the transplanted precursor cells,
- a functional immune system of the xenogenic species, which is restricted to the transgenic MHC class I and/or MHC class II molecules solely,
- a functional syngenic tissue of the xenogenic species,
- a lack of functional murine T lymphocytes, B lymphocytes and NK cells, and
- a lack of murine MHC class I and MHC class II molecules cell surface expression.

In another preferred embodiment of the preceding uses, the tissue is selected from the group consisting of: hepatic, nervous, adipose, cardiac, chondrocytic, endothelial, pancreatic, muscle and skin tissues.

The invention relates also to a method of studying the immunopathogenesis of a tissue-specific disease, *in vivo,* characterized in that it comprises the steps of:
a) inducing a pathology in the syngenic tissue of the multichimeric mouse as defined above, and
b) analysing the immune response to the pathological tissue, into the multichimeric mouse, by any appropriate means.

According to a preferred embodiment of said method, step a) is performed by inoculating a pathogenic microorganism to the mouse chimera, by any appropriate means.

Pathogenic microorganisms include with no limitation: bacteria, fungi, viruses, parasites and prions. Bacteria include for example: *C. diphtheriae, B.pertussis, C. tetani, H. influenzae, S. pneumoniae, E. Coli, Klebsiella, S. aureus, S. epidermidis, N. meningiditis, B. anthracis,* Listeria, *Chlamydia trachomatis* and *pneumoniae,* Rickettsiae, Group A Streptococcus, Group B Streptococcus, *Pseudomonas aeruginosa,* Salmonella, Shigella, Mycobacteria *(Mycobacterium tuberculosis)* and Mycoplasma. Viruses include for example: Polio, Mumps, Measles, Rubella, Rabies, Ebola, Hepatitis A, B, C, D and E, Varicella Zoster, Herpes simplex types 1 and 2, Parainfluenzae, types 1, 2 and 3 viruses, Human Immunodeficiency Virus I and II, , RSV, CMV, EBV, Rhinovirus, Influenzae virus A and B, Adenovirus, Coronavirus, Rotavirus and Enterovirus. Fungi include for example: *Candida sp. (Candida albicans).* Parasites include for example: Plasmodium *(Plasmodium falciparum), Pneumocystis carinii,* Leishmania, and Toxoplasma.

According to another preferred embodiment of said method, step a) is performed by inoculating an inductor of a tumor or an auto-immune disease to the mouse chimera, by any means.

Inductors of tumors or auto-immune diseases are well-known to those of ordinary skill in the art. For example, streptozotocine may be used to induce auto-immune diabetes, and DSS (Dextran sulfate sodium) may be used to induce Inflammatory Bowel Disease (IBD; Shintani et al., General Pharmacology, 1998, 31:477-488). Urethane may also be used to induce lung adenocarcinoma (Steraman et al., Am. J. Pathol., 2005, 167:1763-1775)

According to another preferred embodiment of said method, step b) comprises assaying for the presence of a humoral response, a T-helper cell response or a T-cytotoxic cell response to an antigen which is expressed in said pathological tissue.

The presence of an immune response to the antigen is assayed by any technique well-known in the art.

The presence of a humoral response to the antigen is assayed by measuring, either the titer of antigen-specific antibodies from the sera of murine hosts, by ELISA, or the number of antibody secreting cells, by ELISPOT.

The presence of a T-helper cell response to the antigen is assayed by an *in vitro* T cell proliferation assay, and ELISPOT or intracellular staining and analysis by flow cytometry, for detection of cytokine production.

The presence of a T-cytotoxic cell response to the antigen is assayed by a CTL assay *in vitro* (Cr⁵¹ release) or *in vivo* (Barber et al., J. Immunol., 2003, 171:27-31)..

The invention relates also to a method of screening immunotherapeutic agents or vaccines *in vivo,* characterized in that it comprises the steps of:
- administering an immunotherapeutic agent or a vaccine to the multichimeric mouse as defined above, by any appropriate means,
- inducing a pathology, in the syngenic tissue of the multichimeric mouse, and
- assaying for the presence of an immunoprotective effect of the vaccine or a therapeutic effect of the immunotherapeutic agent in the treated mouse, by comparison with the control (untreated mouse).

According to a preferred embodiment of the preceding methods, the disease is selected from the group consisting of: cancers, auto-immune diseases, and infectious diseases. The cancer may be a solid-tumour or a leukaemia. The auto-immune disease is for example an auto-immune diabetes. The infectious disease may be advantageously selected from the group consisting of: viral hepatitis, malaria, AIDS, Kreutzfeld-Jacob disease and EBV-associated cancers.

The method of the invention may be used for mapping antigens, for screening new antigens and immunotherapeutic drugs, as well as for evaluating the immunogenicity of different antigen preparations for use as human vaccine and the efficiency of different drugs for use as immunotherapeutic in human.

The practice of the present invention will employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Current Protocols in Molecular Biology (Frederick M. AUSUBEL, 2000, Wiley and son Inc, Library of Congress, USA); Molecular Cloning: A Laboratory Manual, Third Edition, (Sambrook et al, 2001, Cold Spring Harbor, New York: Cold Spring Harbor Laboratory Press); Oligonucleotide Synthesis (M. J. Gait ed., 1984); Mullis et al. U.S. Pat. No. 4,683,195; Nucleic Acid Hybridization (B. D. Harries & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the series, Methods In ENZYMOLOGY (J. Abelson and M. Simon, eds.-in-chief, Academic Press, Inc., New York), specifically, Vols.154 and 155 (Wu et al. eds.) and Vol. 185, "Gene Expression Technology" (D. Goeddel, ed.); Gene Transfer Vectors For Mammalian Cells (J. H. Miller and M. P. Calos eds., 1987, Cold Spring Harbor Laboratory); Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986); and Manipulating the Mouse Embryo, (Cold Spring Harbor Laboratory Press, Cold Spring Harbor, N.Y., 1986).

The transgenic mouse which is used in the present invention may be produced by successive crossing of mice carrying one or more mutation(s)/transgene(s) of interest as defined above, and screening of the progenies until double mutants and double transgenics are obtained. They are advantageously produced by crossing a H-2 class I-/class II-KO immunodeficient mice, with a HLA-I+ and/or a HLA-II+ transgenic mouse as defined above. Preferably, said H-2 class I-/class II- KO immunodeficient mice have a Rag2^{-/-}, γ_{c}^{-/-}, β₂m^{-/-}, I-Aβ^{b-/-} genotype.

Based on the disclosure herein, additional MHC class I/MHC class II-transgenic, H-2 class I/class II-KO immunodeficient mice can be constructed by using conventional homologous recombination techniques. For example, additional MHC class I transgenic and additional MHC class II-transgenic may be constructed using conventional homologous recombination techniques. These transgenics may be crossed with H-2 class I-/class II-KO immunodeficient mice as defined above.

"Homologous recombination" is a general approach for targeting mutations to a preselected, desired gene sequence of a cell in order to produce a transgenic animal (Mansour et al., Nature, 1998, 336:348-352; Capecchi, M. R., Trends Genet., 1989, 5:70-76; Capecchi, M. R., Science, 1989, 244:1288-1292; Capecchi et al., In: Current Communications in Molecular Biology, Capecchi, M. R. (ed.), Cold Spring Harbor Press, Cold Spring Harbor, N.Y. (1989), pp. 45-52; Frohman et al., Cell, 1989, 56:145-147). It is now feasible to deliberately alter any gene in a mouse (Capecchi, M. R., Trends Genet., 1989, 5:70-76 ; Frohman et al., Cell, 1989, 56:145-147). Gene targeting involves the use of standard recombinant DNA techniques to introduce a desired mutation into a cloned DNA sequence of a chosen locus. In order to utilize the "gene targeting" method, the gene of interest must have been previously cloned, and the intron-exon boundaries determined. The method results in the insertion of a marker gene (e.g., an nptll gene) into a translated region of a particular gene of interest. Thus, use of the gene targeting method results in the gross destruction of the gene of interest. Significantly, the use of gene targeting to alter a gene of a cell results in the formation of a gross alteration in the sequence of that gene. That mutation is then transferred through homologous recombination to the genome of a pluripotent, embryo-derived stem (ES) cell. The altered stem cells are microinjected into mouse blastocysts and are incorporated into the developing mouse embryo to ultimately develop into chimeric animals. In some cases, germ line cells of the chimeric animals will be derived from the genetically altered ES cells, and the mutant genotypes can be transmitted through breeding.

The chimeric or transgenic animals which are used in the present invention may be prepared by introducing one or more DNA molecules into a mouse cell, which may be a mouse pluripotent precursor cell, such as a mouse ES cell, or equivalent (Robertson, E. J., In: Current Communications in Molecular Biology, Capecchi, M. R. (ed.), Cold Spring Harbor Press, Cold Spring Harbor, N.Y. (1989), pp. 39-44). The pluripotent (precursor or transfected) cell can be cultured *in vivo* in a manner known in the art (Evans et al., Nature, 1981, 292:154-156) to form a chimeric or transgenic animal. Any ES cell can be used in accordance with the present invention. It is, however, preferred to use primary isolates of ES cells. Such isolates can be obtained directly from embryos, such as the CCE cell line disclosed by Robertson, E. J. (In: Current Communications in Molecular Biology, Capecchi, M R. (ed), Cold Spring Harbor Press, Cold Spring Harbor, N.Y. (1989), pp. 39-44*),* or from the clonal isolation of ES cells from the CCE cell line (Schwartzberg et al., Science, 1989, 246:799-803, which reference is incorporated herein by reference). Such clonal isolation can be accomplished according to the method of E. J. Robertson (In: Teratocarcinomas and Embryonic Stem Cells: A Practical Approach, (E. J Robertson, Ed.), IRL Press, Oxford, 1987*),* which reference and method are incorporated herein by reference. The purpose of such clonal propagation is to obtain ES cells, which have a greater efficiency for differentiating into an animal. Clonally selected ES cells are approximately 10-fold more effective in producing transgenic animals than the progenitor cell line CCE. For the purposes of the recombination methods of the present invention, clonal selection provides no advantage. An example of ES cell lines, which have been clonally derived from embryos, are the ES cell lines, ABI (hprt⁺) or AB2.1 (hprt"). The ES cells are preferably cultured on stromal cells (such as STO cells (especially SNC4 STO cells) and/or primary embryonic fibroblast cells) as described by E. J. Robertson (In: Teratocarcinomas and Embryonic Stem Cells: A Practical Approach, (E. J Robertson, Ed., IRL Press, Oxford, 1987, pp 71-112*),* which reference is incorporated herein by reference. Methods for the production and analysis of chimeric mice are disclosed by Bradley, A. (In: Teratocarcinomas and Embryonic Stem Cells: A Practical Approach, (E. J Robertson, Ed.), IRL Press, Oxford, 1987, pp 113-151), which reference is incorporated herein by reference. The stromal (and/or fibroblast) cells serve to eliminate the clonal overgrowth of abnormal ES cells. Most preferably, the cells are cultured in the presence of leukocyte inhibitory factor ("lit") (Gough et al., Reprod. Fertil. Dev., 1989, 1:281-288; Yamamori, Y. et al., Science, 1989, 246:1412-1416. Since the gene encoding lif has been cloned (Gough, N. M. et al., 1989, Reprod. Fertil. Dev. 1:281-288), it is especially preferred to transform stromal cells with this gene, by means known in the art, and to then culture the ES cells on transformed stromal cells that secrete lif into the culture medium.

In addition to the preceding features, the invention further comprises other features which will emerge from the description which follows, which refers to examples illustrating the method according to the invention, as well as to the appended drawings in which:

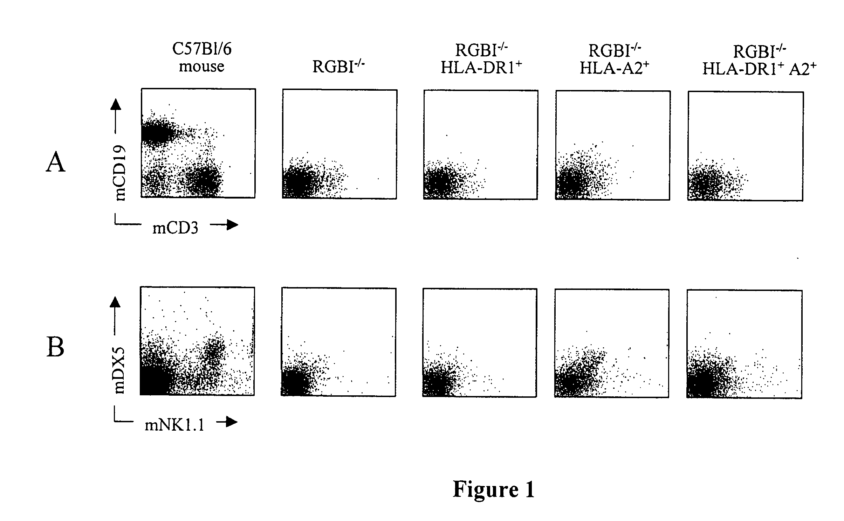

- figure 1 illustrates the absence of T, B and NK T cells in the transgenic mice. Splenocytes from C57B1/6 mice, Rag^{-/-} γc^{-/-}ß2m^{-/-} IAβ^{b-/-} (referred as RGBI^{-/-}), Rag^{-/-}γc^{-/-}ß2m^{-/-} IAβ^{b-/-} HLA-DR1⁺, Rag^{-/-}γc^{-/-}ß2m^{-/-} IAβ^{b-/-} HLA-A2⁺, Rag^{-/-} γ c^{-/-}ß2m^{-/-} IAβ^{b-/-} HLA-DR1⁺ HLA-A2⁺ mice were stained for murine CD19 and murine CD3 (A) and murine NK1.1 and murine DX5 (B). The panels are representative of three mice of each group and two human donors.

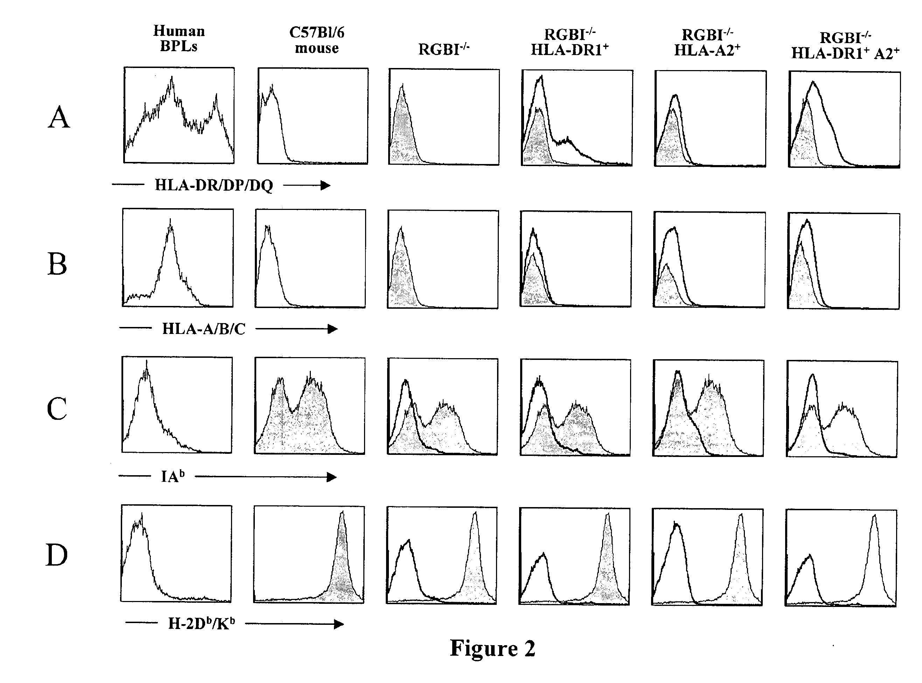

- figure 2 illustrates the analysis of MHC molecule expression in the transgenic mice. Human PBLs and splenocytes from C57B1/6 mice, Rag^{-/-} γc^{-/-}ß2m^{-/-} IAβ^{b-/-} (referred as RGBI^{-/-}), Rag^{-/-} γc^{-/-}ß2m^{-/-} IAβ^{b-/-} HLA-DR1⁺, Rag^{-/-} γc^{-/-}ß2m^{-/-} IAβ^{b-/-} HLA-A2⁺, Rag^{-/-} γc^{-/-}ß2m^{-/-} IAβ^{b-/-} HLA-DR1⁺ HLA-A2⁺ mice were stained for HLA-DR/DP/DQ (A), HLA-A/B/C (B), IA^{b}(C), H-2D^{b}/K^{b} (D).

For Rag^{-/-} γc^{-/-}ß2m^{-/-} IAβ^{b-/-} HLA-DR1⁺, Rag^{-/-} γc^{-/-}ß2m^{-/-} IAβ^{b-/-} HLA-A2⁺, Rag^{-/-} γc^{-/-}ß2m^{-/-} IAβ^{b-/-} HLA-DR1⁺ HLA-A2⁺ mice, HLA-DR/DP/DQ (A), and HLA-A/B/C (B) expression profile (opened histograms) are shown together with that obtained for Rag^{-/-} γc^{-/-}ß2m^{-/-} IAβ^{b-/-} control mice (filled histograms).

For Rag^{-/-}γc^{-/-}ß2m^{-/-} IAβ^{b-/-}, Rag^{-/-}γc^{-/-}ß2m^{-/-} IAβ^{b-/-} HLA-DR1⁺, Rag^{-/-} γc^{-/-}ß2m^{-/-} IAβ^{b-/-} HLA-A2⁺, Rag^{-/-}γc^{-/-}ß2m^{-/-} IAβ^{b-/-} HLA-DR1⁺ HLA-A2⁺ mice, IAβ^{b} (C) and H-2D^{b}/K^{b} (D). Expression profiles (opened histograms) are shown together with that obtained for C57B1/6 control mice (filled histograms).

The panels are representative of three mice of each group and two human donors.

### Example 1: Transgenic mice production

### 1) Material and methods

### a) Mice

The transgenic mice were produced by crossing of different mice strains:
- Rag2^{-/-}, γ_{c}^{-/-} (129 background), obtained by crossing Rag2^{-/-} mice (Takeda et al., Immunity, 1996, 5, 217-228) with γ_{c}^{-/-} mice (DiSanto et al., P.N.A.S., 1995, 92, 377-381).
- β₂m^{-/-} (C57B1/6 background), described in Zijlstra et al., Nature, 1990, 344, 742-746.
- I-Aβ^{b -/-} (C57Bl/6 background), described in Takeda et al., Immunity, 1996, 5, 217-228.
- HLA-DR1 transgenic mice (DR1⁺ , FVB background), expressing the DR1 molecule encoded by the HLA-DRA*0101 and HLA-DRB1*0101 gene, described in Altmann et al., J. Exp. Med., 1995, 181, 867-875.
- HLA-A2.1 transgenic mice, expressing a chimeric monochain (α1-α2 domains of HLA-A2.1 (encoded by HLA-A*0201 gene), α3 to cytoplasmic domains of H-2 D^{b}, linked at its N-terminus to the C terminus of human β2m by a 15 amino-acid peptide linker), described in Pascolo et al., J. Exp. Med., 1997, 185, 2043-2051.

More precisely, the Rag2^{-/-}, γ_{c}^{-/-} mice (129 background) were crossed with 1-Aβ^{b -/-} (C57B1/6 background) and the F1 progeny were crossed successively with β₂m^{-/-} (C57B1/6 background), HLA-DR1 transgenic mice (DR1⁺, FVB background) and HLA-A2.1 transgenic mice. I-Aα^{b+} (essential for murine MHC class II ^{-/-} phenotype) and C5 ^{-/-} progenies (both 129 and FVB strains are constitutively C5^{-/-}). were selected from each crossing.

### b) Genotyping

The genotype of each progeny was determined by PCR on tail-DNA using standard protocols and the following primers for the different genes:

**Table I: Primers used for genotyping**

| Gene | Primer sequences (SEQ ID NO : 1 to 22) |
|---|---|
| Rag2 | RagA : GGG AGG ACA CTC ACTTGC CAG TA |
| | RagB : AGT CAG GAG TCT CCATCT CAC TGA |
| | neo :CGG CCG GAG AAC CTGCGT GCA A |
| γc | GCF 1 : AGC TCC AAG GTC CTC ATG TCC AGT |
| | GCF2 : GTG TAC TGT TGG TTGGAA CGG TGA G |
| | GCR : GGG GAG GTT AGC GTCACT TAG GAC |
| 1-Aα^{b} | Ea5' : AGT CTT CCC AGC CTTCAC ACT CAG AGG TAC |
| | EA3' : CAT AGC CCC AAA TGTCTG ACC TCT GGA GAG |
| I-Aβ^{b} | Iaß^{b}f : CCG TCC GCA GGG CATTTC GTG TA |
| | Iaß^{b}r : AGG ATC TCC GGC TGG CTGTTC |
| β2m | β2M fwd : AAT GGG AAG CCG AACATA CTG AAC |
| | β2M rvs : GTC ATG CTT AAC TCTGCA GGC GTA |
| | neo : CTT AAT ATG CGA AGTGGA CCT GGG |
| HLA-DR1 | DR1f : TTC TTC AAC GGG ACGGAG CGC GTG |
| | DR 1r : CTG CAC TGT GAA GCTCTC ACC AAC |
| HLA-A2 | B2hAS 1332 : GGA TGA CGT GAG TAAACC TGA ATC TTT GGAGTA CGC |
| | PROMA2S964 : CAT TGA GAC AGA GCGCTT GGC ACA GAA GCAG |
| C5 | C5-20 : TGG CAT TTC AGA CAATGG TAG |
| | C5-21 GTG TAT CAG CAA CACATA TAC |
| | 945 : AAG CAC TAG CAT CTCAAA CAA |
| | C5pos : CAA CAA CTG GAA CTGCAT AC |
| | CSneg : ACA ACA ACT GGA ACTGCA CT |

### c) Flow cytometry analysis

Human PBLs, splenocytes of C57B1/6, Rag2^{-/-}, γ_{c}^{-/-}, β₂m^{-/-}, I-Aβ^{b -/-}, HLA-A2^{+/+}, HLA-DR1^{+/+}, Rag2^{-/-}, γ_{c}^{-/-}, β₂m^{-/-}, I-Aβ^{b*-*/}*⁻,* HLA-A2^{+/+}, Rag2^{-/-}, γ_{c}^{-/-}, β₂m^{-/-}, I-Aβ^{b -/-}, HLA-DR1^{+/+}, and Rag2^{-/-}, γ_{c}^{-/-}, β₂m^{-/-}, I-Aβ^{b-/-} mice were dilacerated and incubated with anti-FcR antibodies. The cells where then stained in PBS containing 0,05 % azide, 2% FCS, and anti-HLA-DR/DP/DQ, anti-HLA-A/B/C, anti-H-2 IA^{b}, anti-H-2D^{b} + anti-K^{b}, anti-mouse CD19, anti-mouse CD3, anti-mouse NK1.1 monoclonal antibodies (mAbs) conjugated with appropriate fluorochrome. After staining, cells were washed twice and resuspended in the same buffer. Labelling was analyzed on a LSR flowcytometer with CellQuest software.

### 2) Results

Four mice strains were obtained :
- Rag2^{-/-}, γ_{c}^{-/-}, β₂m^{-/-}, I-Aβ^{b -/-} , HLA-A2^{+/+}, HLA-DR1^{+/+}
- Rag2^{-/-}, γ_{c}^{-/-}, β₂m^{-/-}, I-Aβ^{b-/-}, HLA-A2^{+/+},
⁻Rag2^{-/-}, γ_{c}^{-/-}, β₂m^{-/-}, I-Aβ^{b-/-}, HLA-DR1^{+/+}, and
-Rag2^{-/-},γ_{c}^{-/-},β₂m^{-/-},I-Aβ^{b-/}-,

The absence of murine T, B (Figure 1A) and NK cells (Figure 1B) in the four types of mice was evaluated by fluorescence-activated cell sorting. Cell surface expression of the HLA-A2.1, H-2 K^{b}/D^{b}, HLA-DR1 and H-2 IA^{b} molecules was evaluated by flow cytometry Cell surface expression of endogenous H-2 class I and class II molecules was absent in the four mice (Figure 2C and 2D). A similar level of HLA-A2.1 expression (Figure 2B) was observed in HLA A2.1 /HLA-DR1 transgenic H-2 class I-/class II-KO immunodeficient mice and HLA A2.1 transgenic H-2 class I-/class II-KO immunodeficient mice, while HLA-A2.1 was absent in HLA-DR1 transgenic H-2 class I-/class II-KO mice and H-2 class I-/class II-KO immunodeficient mice. A similar level of HLADR1 expression (Figure 2A) was observed in HLA A2. 1-/HLA-DR1-transgenic H-2 class I-/class II-KO immunodeficient mice and HLA-DR1-transgenic H-2 class I-/class II-KO immunodeficient mice, whereas no expression was detected in HLA A2.1-transgenic H-2 class I-/class II-KO immunodeficient mice and H-2 class I-/class II-KO immunodeficient mice. HLA-DR1 and -A2 molecule are, however, lower in transgenic mice than in human PBLs.

### Example 2: Human/mouse multichimera production and characterization

CD34+ cells were derived from human embryonic stem cells according to the procedure described in Vodyanik et al., Blood, 2005, 105, 617-626. Briefly, ES cells were co-cultured for 6-9 days on 4 day-old confluent OP-9 murine bone marrow stromal cell line. CD34+ percentage was determined by flow cytometry using an anti-human CD34 PE conjugated monoclonal antibodies (mAbs; BECTON DICKINSON). Single cell suspension was then prepared by treatment by collagenase IV (INVITROGEN) and CD34+ cells were magnetically sorted using Direct CD34 progenitor Cell Isolation Kit (MILTENYI BIOTECH) as recommended by the manufacturer, and processed on an Automacs machine (MILTENYI BIOTECH).

Hepatocyte progenitors were derived from human embryonic stem (ES) cells, according to the method described in N; Lavon et al., Differenciation, 2004, 72:230-238. Briefly, a reporter gene (green fluorescence protein) under the regulation of an hepatocyte-specific promoter, was introduced into human ES cells. Upon *in vitro* differentiation (formation of Embryonic bodies), green fluorescent cells were Facs sorted (FacsAria or MoFlow Facs sorters from COULTER). The cells function (urea synthesis) an gene expression (AFP, ALB, APOA4, APOB, APOH, FGA, FGG, FGB, AAT) was analyzed.

13-islet progenitors were derived from human ES cells, according to a method which is a modified version of the method described in G.K.C. Brolen et al., Diabetes, 2005, 54:2867-2874. Briefly, a reporter gene (green fluorescence protein) under the regulation of a by a ß-islet-specific promoter (Pdxl or Foxa2 or Isl1) was introduced into human ES cells. Human ES were allowed to differentiate *in vitro* (formation of Embryonic bodies), for up to 34 days. When Foxa2⁺, Pdx1⁺, Isl1⁺ cells became numerous at the peripheral areas of dhES cell colonies, as assessed by immunofluorescence analysis, fluorescent green cells were Facs sorted (FacsAria or MoFlow Facs sorters from COULTER).

At day of birth, newborn mice were irradiated in a 4 hour interval with 2x2 Gy from a Cesium 137 source at 4 Gy/min., a dose that was titrated to be sub lethal. At six hours post irradiation, both hematopoietic (CD34⁺ cells; 1 to 2.10⁶ in 25 µl PBS) and hepatic non-hematopoietic progenitor human green cells (1 to 2.10⁶ in 25 µl PBS) cells were co-injected into the liver (i.h.) using a 29-gauge needle.

Three months after transplantation, mice were bled from tail vein, to obtain peripheral blood cells and plasma. Some of the mice where then sacrificed, single cell suspensions from organs were prepared. The cells were analyzed by flowcytometry (LSR or facsanto, COULTER, BD), using antibodies against human CD45, CD19, CD3, CD4 or CD8, CD11c conjugated with an appropriate fluorochrome (BD, COULTER). For liver engraftment, the presence of green cells was assessed by flowcytometry together with function (metabolic and detoxifying enzymes).

### Example 3 : Use of the multichimeric mouse to study the immunopathogenesis of a disease

The human/mouse chimera are used as a model for HIV infection and in particular to study the role of CD8 T cells in the control and/or pathogenesis of this infection.

Human/mouse chimera already reconstituted by human immune system, as described in example 2, were injected intraperitoneally with either HIV virus alone (viral strains or primary isolates) or infected PHA-blastic allogenic T cells. Blood samples were taken at different time points post-infection to monitor both the percentage of human CD4 and CD8 T cells by flow cytometry, and the plasmatic viral load (proviral DNA and viral RNA).

The anti-viral T cell responses in infected chimera was evaluated and compared with the response obtained by T cells from non-infected chimera, in the following assays:
- the expansion of anti-viral T cells from chimera spleen and lymph nodes was measured by flow cytometry using tetramers of different HLA-DR1- or HLA-A2-restricted HIV peptide complexes,
- the production of γIFN, αTNF and IL-2 was assessed after *in vitro* restimulation of spleen and lymph nodes T cells by different HLA-DR1- or HLA-A2-restricted HIV peptides, using ELISPOT or flow cytometry (intracellular staining), and
- the anti-viral cytotoxicity was measured by *in vitro* ⁵¹Cr release assay and *in vivo* injection of CFSE-labeled syngenic B cell targets loaded or non-loaded with different HLA-A2-restricted HIV peptides.

The role of CD8 T cells during HIV infection was evaluated by CD8 T cells depletion and infusion:
- infected chimera were depleted of CD8 T cells using injection of anti-human CD8 antibodies. The depletion was checked by flow cytometry from blood samples. The percentage of CD4 T cells and the splenic and lymph node viral loads were then compared between CD8-depleted and CD4-replete HIV-infected chimera, and
- syngenic CD8 T cells from HIV-infected chimera were adoptively transferred into CD8 T cell-depleted HIV infected chimera. The percentage of CD4 T cells and the splenic and lymph node viral loads were then compared between CD8-injected and CD8-non-injected HIV-infected chimera.

The role of CD8 T cells on HIV susceptibility was evaluated in CD8-depleted and CD8-replete chimera, injected by HIV. Productive infection was assessed and compared between CD8-depleted and CD8-replete chimera at different time points post-injection. Therefore, blood, thymus, spleen and lymph node cells were tested for the presence of virus by RT-PCR (viral load) and pro-virus by PCR (proviral load), before and after PHA-stimulation.

## Claims

1. A method of producing a multichimeric mouse, **characterized in that** it comprises the step of: transplanting syngenic precursor cells of a xenogenic species into a transgenic mouse, by any appropriate means, wherein:
a) the syngenic precursor cells comprise hematopoietic and non-hematopoietic precursors, and
b) the transgenic mouse has a phenotype comprising :
b₁) a deficiency for murine T lymphocytes, B lymphocytes and NK cells,
b₂) a deficiency for murine MHC class I and MHC class II molecules, and
b₃) a functional MHC class I transgene and/or a functional MHC class II transgene from the same species as the precursor cells.

2. The method according to claim 1, **characterized in that** the precursor cells are derived from stem cells.

3. The method according to anyone of claim 1 or claim 2, **characterized in that** the precursor cells are from donors of different MHC haplotypes.

4. The method according to claim 3, **characterized in that** the different haplotypes reflect the genetic variability of the xenogenic species.

5. The method according to anyone of claims 1 to 4, **characterized in that** the precursor cells are human precursor cells.

6. The method according to anyone of claims 1 to 5, **characterized in that** the hematopoietic precursor cells are human CD34⁺ cells.

7. The method according to anyone of claims 1 to 6, **characterized in that** the non-hematopoietic precursor cells are selected from the group consisting of: hepatocyte, neurone, adipocyte, myocyte, chondrocyte or melanocyte precursors, and endothelial, glial or pancreatic cells precursors.

8. The method according to anyone of claims 1 to 7, **characterized in that** the hematopoietic or non-hematopoietic precursors are genetically modified by an oligonucleotide or a polynucleotide of interest.

9. The method according to anyone of claims 1 to 8, **characterized in that** the hematopoietic precursors and non-hematopoietic precursors are transplanted simultaneously.

10. The method according to anyone of claims 1 to 8, **characterized in that** the hematopoietic precursors and non-hematopoietic precursors are transplanted sequentially.

11. The method according to anyone of claims 1 to 10, **characterized in that** the hematopoietic precursors and non-hematopoietic precursors are transplanted in the same site of the mouse.

12. The method according to anyone of claims 1 to 10, **characterized in that** the hematopoietic precursors and non-hematopoietic precursors are transplanted in a different site of the mouse

13. The method according to anyone of claims 1 to 12, **characterized in that** the deficiency of the transgenic mouse as defined in b₁), is associated with a deficient Rag2 gene and a deficient common receptor γ chain gene.

14. The method according to anyone of claims 1 to 13, **characterized in that** the transgenic mouse murine MHC class I molecules deficiency, is associated with a deficient β2-microglobulin gene.

15. The method according to anyone of claims 1 to 14, **characterized in that** the transgenic mouse murine MHC class II deficiency, is associated with a deficient H-2^{b}-Aβ gene.

16. The method according to anyone of claims 1 to 15, **characterized in that** the transgenic mouse xenogenic MHC class I and/or class II transgenes are human HLA class I and/or HLA class II transgenes.

17. The method according to claim 16, **characterized in that** the HLA class I transgene is an HLA-A2 transgene and the HLA class II transgene is an HLA-DR1 transgene.

18. The method according to claim 17, **characterized in that** the transgenic mouse has a genotype selected from the group consisting of :
-Rag2^{-/-}, γ_{c}^{-/-}, β₂m^{-/-}, I-Aβ^{b-/-}, HLA-A2^{+/+}, HLA-DR1^{+/+},
-Rag2^{-/-}, γ_{c}^{-/-}, β₂m^{-/-}, I-Aβ^{b-/-}, HLA-A2^{+/+,} and
⁻Rag2^{-/-}, γ_{c}^{-/-}, β₂m^{-/-}, I-Aβ^{b-/-}, HLA-DR1^{+/+}.

19. The method according to anyone of claims 1 to 18, **characterized in that** the transgenic mouse further comprises a deficiency for the C5 protein of complement.

20. Use of a multichimeric mouse obtainable by the method according to anyone of claims 1 to 19, to study tissue differentiation *in vivo.*

21. Use of a multichimeric mouse obtainable by the method according to anyone of claims 1 to 19, to study the immununopathogenesis of a tissue-specific disease.

22. The use according to claim 20 or claim 21, **characterized in that** the multichimeric mouse comprises:
- functional transgenic-MHC class I and/or MHC class II molecules of the xenogenic species,
- a functional immune system of the xenogenic species, which is restricted to the transgenic MHC class I and/or MHC class II molecules solely,
- a functional syngenic tissue of the xenogenic species,
- a lack of functional murine T lymphocytes, B lymphocytes and NK cells, and
- a lack of murine MHC class I and MHC class II molecules cell surface expression.

23. The use according to anyone of claims 20 to 22, **characterized in that** the multichimeric mouse is a human/mouse chimera obtained by human precursor cells transplantation.

24. The use according to anyone of claims 20 to 23, **characterized in that** the tissue is selected from the group consisting of: hepatic, nervous, adipose, cardiac, chondrocytic, endothelial, pancreatic, muscle and skin tissues.

25. A method of studying the immunopathogenesis of a tissue-specific disease, *in vivo,* **characterized in that** it comprises the steps of:
a) inducing a pathology, in the syngenic tissue of the multichimeric mouse as defined in claim 22 or claim 23, and
b) analysing the immune response to the pathological tissue, into the multichimeric mouse, by any appropriate means.

26. The method according to claim 25, **characterized in that** step a) is performed by inoculating a pathogenic microorganism to the mouse chimera, by any appropriate means.

27. The method according to claim 25, **characterized in that** step a) is performed by inoculating an inductor of a tumor or an auto-immune disease to the mouse chimera, by any appropriate means.

28. The method according to anyone of claims 25 to 27, **characterized in that** step b) comprises assaying for the presence of a humoral response, a T-helper cell response or a T-cytotoxic cell response to an antigen which is expressed in said pathological tissue.

29. A method of screening immunotherapeutic agents or vaccines *in vivo,* **characterized in that** it comprises the steps of:
- administering an immunotherapeutic agent or a vaccine to the multichimeric mouse as defined in anyone of claims 21 to 23, by any appropriate means,
- inducing a pathology, in the syngenic tissue of the multichimeric mouse, and
- assaying for the presence of an immunoprotective effect of the vaccine or a therapeutic effect of the immunotherapeutic agent in the treated mouse, by comparison with the untreated mouse control.

30. The method according to anyone of claims 25 to 29, **characterized in that** said disease is selected from the group consisting of : cancers, auto-immune diseases, and infectious diseases.

31. The method according to claim 30, **characterized in that** said auto-immune disease is diabetes.

32. The method according to claim 30, **characterized in that** said infectious disease is selected from the group consisting of: viral hepatitis, malaria, AIDS, Kreutzfeld-Jacob disease and EBV-associated cancers.


SEQUENCE LISTING

<110>  INSTITUT PASTEUR

<120>  METHOD OF PRODUCING A MULTICHIMERIC MOUSE AND APPLICATIONS TO
       STUDY THE IMMUNOPATHOGENESIS OF HUMAN TISSUE-SPECIFIC PATHOLOGIES

<130>  226ep130

<160>  22

<170>  PatentIn version 3.3

<210>  1
<211>  23
<212>  DNA
<213>  ARTIFICIAL SEQUENCE

<220>
<223>  PRIMER

<400>  1
gggaggacac tcacttgcca gta                                              23


<210>  2
<211>  24
<212>  DNA
<213>  ARTIFICIAL SEQUENCE

<220>
<223>  PRIMER

<400>  2
agtcaggagt ctccatctca ctga                                             24


<210>  3
<211>  22
<212>  DNA
<213>  ARTIFICIAL SEQUENCE

<220>
<223>  PRIMER

<400>  3
cggccggaga acctgcgtgc aa                                               22


<210>  4
<211>  24
<212>  DNA
<213>  ARTIFICIAL SEQUENCE

<220>
<223>  PRIMER

<400>  4
agctccaagg tcctcatgtc cagt                                             24


<210>  5
<211>  25
<212>  DNA
<213>  ARTIFICIAL SEQUENCE

<220>
<223>  PRIMER

<400> 5
gtgtactgtt ggttggaacg gtgag 25

<210> 6
<211> 24
<212> DNA
<213> ARTIFICIAL SEQUENCE

<220>
<223> PRIMER

<400> 6
ggggaggtta gcgtcactta ggac 24

<210> 7
<211> 30
<212> DNA
<213> ARTIFICIAL SEQUENCE

<220>
<223> PRIMER

<400> 7
agtcttccca gccttcacac tcagaggtac 30

<210> 8
<211> 30
<212> DNA
<213> ARTIFICIAL SEQUENCE

<220>
<223> PRIMER

<400> 8
catagcccca aatgtctgac ctctggagag 30

<210> 9
<211> 23
<212> DNA
<213> ARTIFICIAL SEQUENCE

<220>
<223> PRIMER

<400> 9
ccgtccgcag ggcatttcgt gta 23

<210> 10
<211> 21
<212> DNA
<213> ARTIFICIAL SEQUENCE

<220>
<223> PRIMER

<400> 10
aggatctccg gctggctgtt c 21

<210> 11
<211> 24
<212> DNA

<213>  ARTIFICIAL SEQUENCE

<220>
<223>  PRIMER

<400>  11
aatgggaagc cgaacatact gaac                                              24


<210>  12
<211>  24
<212>  DNA
<213>  ARTIFICIAL SEQUENCE

<220>
<223>  PRIMER

<400>  12
gtcatgctta actctgcagg cgta                                              24


<210>  13
<211>  24
<212>  DNA
<213>  ARTIFICIAL SEQUENCE

<220>
<223>  PRIMER

<400>  13
cttaatatgc gaagtggacc tggg                                              24


<210>  14
<211>  24
<212>  DNA
<213>  ARTIFICIAL SEQUENCE

<220>
<223>  PRIMER

<400>  14
ttcttcaacg ggacggagcg cgtg                                              24


<210>  15
<211>  24
<212>  DNA
<213>  ARTIFICIAL SEQUENCE

<220>
<223>  PRIMER

<400>  15
ctgcactgtg aagctctcac caac                                              24


<210>  16
<211>  36
<212>  DNA
<213>  ARTIFICIAL SEQUENCE

<220>
<223>  PRIMER

<400>  16
ggatgacgtg agtaaacctg aatctttgga gtacgc                                 36

<210>  17
<211>  31
<212>  DNA
<213>  ARTIFICIAL SEQUENCE

<220>
<223>  PRIMER

<400>  17
cattgagaca gagcgcttgg cacagaagca g                                    31


<210>  18
<211>  21
<212>  DNA
<213>  ARTIFICIAL SEQUENCE

<220>
<223>  PRIMER

<400>  18
tggcatttca gacaatggta g                                               21


<210>  19
<211>  21
<212>  DNA
<213>  ARTIFICIAL SEQUENCE

<220>
<223>  PRIMER

<400>  19
gtgtatcagc aacacatata c                                               21


<210>  20
<211>  21
<212>  DNA
<213>  ARTIFICIAL SEQUENCE

<220>
<223>  PRIMER

<400>  20
aagcactagc atctcaaaca a                                               21


<210>  21
<211>  20
<212>  DNA
<213>  ARTIFICIAL SEQUENCE

<220>
<223>  PRIMER

<400>  21
caacaactgg aactgcatac                                                 20


<210>  22
<211>  20
<212>  DNA
<213>  ARTIFICIAL SEQUENCE

<220>
<223>  PRIMER

<400> 22
acaacaactg gaactgcact 20